# EUROPÄISCHE PATENTSCHRIFT

(11) **EP 0 306 690 B1**
(45) Veröffentlichungstag und Bekanntmachung des Hinweises auf die Patenterteilung: **15.09.1993**
(21) Anmeldenummer: 88112202.2
(22) Anmeldetag: 28.07.1988
(51) Int. Cl.: A61F 2/04, A61F 2/06, F16L 11/00

(54) **Glatte oder gefaltete gewirkte Gefaessprothese**
Unruffled or ruffled knitted vessel prosthesis
Prothèse de vaisseaux tissée, lissè ou plissée

(30) Priorität: 07.08.1987 CS 5861/87
(43) Veröffentlichungstag der Anmeldung: 15.03.1989
(73) Patentinhaber: VYZKUMNY USTAV PLETARSKY, CS-658 61 Brno (CS)
(72) Erfinder: Prochazka, Rostislav, Brno (CS); Skrêpek, Jan, Olsany u Vyskova (CS); Pospisil, RNDr Lubomir, Brno (CS); Kramplova, Milada, Dipl.Ing., Brno (CS)
(74) Vertreter: Fritzsche, Thomas M., Dr.

(56) Entgegenhaltungen:
- EP-A- 0 117 072
- GB-A- 2 189 150
- US-A- 4 193 137
- US-A- 4 208 745
- US-A- 4 306 318

## Beschreibung

Die Erfindung betrifft eine implantierbare Textilie in Form einer gewirkten glatten oder gefalteten Gefässprothese gemäß dem Oberbegriff des Anspruch 1, welche in einer Kettenbindung aus synthetischen Fäden als ein glattes oder verzweigtes röhrenartiges Gebilde erZeugt ist. Die angewendeten Fäden können glatt oder texturiert sein, eventuell wird eine Kombination glatter oder texturierter Fäden benutzt. Die Oberfläche der Gefässprothese kann glatt oder schlingenartig sein und das auf der Aussen- und/oder Innenseite. Die Prothese ist aus länglichen Streifen in einer Kettenbindung erzeugt und diese Streifen sind miteinander längs der Kanten verbunden.

Die Gefässprothesen werden für den Bedarf der Rekonstruktionsgefässchirurgie in den geforderten Durchmessern erzeugt, welche der Arteriehelligkeit, eventuell der Venenheligkeit entsprechen. Das bekannte und angewendete Sortiment dieser Implantate in der Gegenwart umfasst ausser den Grundausführungen in der einfachsten Form auch verschiedene Speziallösungen, welche sich für atypische, weniger übliche Eingriffe eignen.

In der letzten Zeit treten neue Typen von Gefässprothesen in den Vordergrund deren Konstruktion auf die Bedingungsoptimalisierung bei der Vorarbeitung, Implantation und hauptsächlich beim Heilungsprozess und der Einwachsung im menschlichen Organismus nach der eigenen Implantation, orientiert ist. In diesem Zusammenhang ist eine erhöhte Aufmerksamkeit und Sorgfalt der Frage rund um die Wanddurchlässigkeit der Gefässprothese gewidmet, hauptsächlich bei Patienten mit dem betroffenen Koagulationssystem.

Aus der Fachmännischen- und Patentliteratur und aus der Praxis sind gewirkte Gefässprothesen röhrchenartiger Form bekannt, welche durch die Kulierwirktechnologie, sowie auch der Kettenwirktechnologie hergestellt werden und welche sich an den chirurgischen Arbeitsplätzen voll bewährt haben, denn sie wurden mit Berücksichtigung zu den neuersten Erkenntnisen von der Hinsicht der Forderungserfüllung auf eine entsprechende endgültige Verwendung dieser Implantate konstruiert. So ist z.B. gemäss der US-PS 3 945 052 eine Gefässprothese bekannt, deren Konstruktion auf die Ausnutzung der verbesserten Blutdurchflussmenge auf Grund länglich durchlaufender Rippen innerhalb des gewirkten Schlauchbandes orientiert ist, das in der Kettenbindung erzeugt wird. Der Schlauch wird zu diesem Zweck in einer Doppelgrundtrikotbindung oder in einer doppelkombinierten Trikot- oder Tuchbindung aus gewöhnlich benutzten synthetischen Fäden, wie z.B. Dacron, gewirkt. Nach der Erfindungsbeschreibung ist ein verbesserter Blutdurchfluss innerhalb des röhrenartigen Implantats durch die Umwendung der fertiggewirkten Schlauchkonstruktion,mit der Innenseite verkehrt, erreicht, wodurch die länglich gelagerten Rippen von der ursprünglich äusseren Oberfläche sich in den Hohlraum des gewirkten Schlauches wenden.

Gleichfalls ist ein Gefässimplantat in einer Kettenbindung bekannt, geschützt durch die US-PS 4 047 252. In diesem Fall geht es um ein gewirktes hohles Aussteifgitter, mit einer beiderseitigen Florabdeckung aus Schlingen ungleicher Länge versehen, aber in einem gegebenen gegenseitigen Verhältnis einer Oberfläche zu anderen. Die Schlingen sind, was die Bindung anbelangt, in dem hohlen Aussteifgitter so befestigt, dass die längeren Schlingen an die äussere Oberfläche durchdringen, während die kürzeren Schlingen einwärts in den Hohlkörper zielen. Das Gitter ist aus glatten synthetischen, z.B. Polyestergarnen gewirkt, wogegen die Schlingen aus texturierten synthetischen z.B. gleichfalls aus Polyestergarnen gewirkt sind.

Eine sehr ähnliche Gefässprothese wie in der oben genannten US-PS 4 047 252 ist Inhalt einer anderen US-PS 4 193 137. Diese Prothese ist ebenfalls Kettenformig gewirkt, z.B. aus Polyestergarnen, wobei die Grundfläche des Gewirkes wiederum aus glatten Fäden und die Schlingen aus texturierten Fäden erzeugt wird. Die Grundfläche des Gewirkes wird durch geschlossene Maschen gebildet und die Texturgarne bilden offene Maschen. Die Prothese ist aus zwei flächenartigen länglichen Streifen, welche miteinander am Rand entlang verbunden sind, wobei jeder von den beiden gewirkten länglichen Streifen mit einem entzweitem Teil gewirkt ist, das bedeutet, dass sich die volle Breite des länglichen Streifens auf der Fertigungsmaschine in zwei Zweige verteilt. Die Kombination dieser beiden Strickgarnarten, was glatte und texturierte Garne sind, ist nach der Beschreibung der sachlichen US-Patentschrift erfolgreich und ermöglicht in einem breiten Bereich die Dichte einer so erzeugten Gefässprothese zu regulieren.

Die beschriebenen und diesen Konstruktionen vorhergehende Lösungen der Gefässprothesen aus den Kettenwirkmaschinen sind im Vergleich zu ähnlichen Produkten aus der Kuliermaschine ein Beitrag in der Entwicklungsreihe der Implantate und das vom Standpunkt ihrer verdrängten Auftrenbarkeit und einer Verdichtungsmöglichkeit im Laufe einer weiteren physikalischen und chemischen Behandlung, das bedeutet also bei der Ausrüstung. Vorteilhaft ist ebenfalls eine praktische Applikation dieser Produkte, die in Anbetracht zu den Gefässprothesen mit der Kulierbindung feste Kanten und eine verminderte Neigung zur Randzerfransung bei der Arbeit des Chirurgen aufweisen.Dieser bekannte Stand der Konstruktionsalternativen der Gefässprothesen in einer Kettenbindung garantiert aber keine genügend erforderliche optimale Einwachsung in das menschliche Gewebe und bietet keine Bedingungen für eine genügende Lenkung und Kontrole der Wanddurchlässigkeit der Prothese, welche eine beträchtliche Wichtigkeit bei Rekonstruktionen und Auswechslungen hauptsächlich defekter Arterien hatt.

Ziel der Erfindung ist deshalb eine vervollkommende Konstruktion der Gefässprothese und Materialzusammensetzung des Implantats mit der Voraussetzung einer wirkungsvollen Regelung der Wanddurchlässigkeit des Erzeugnises so, dass in einem maximalen Masse ein optimaler Heilungsprozess und eine Bildung der Neointimen erzielt wird. Durch den Abfall der Vorgerinnung wird die Operationsausführung beschleunigt und vereinfacht und das Risiko der Frühblutung nach der Operation wird grundsätzlich vermindert. Durch die Lösung laut Erfindung werden gleiche Durchlässigkeitswerte in allen Punkten der Gefässprothese gesichert. Neben dem wird, was die Bindung anbelangt, die kritische Übergangsstelle zwischen dem vollen Durchmesser des Schlauches und dem einzelnen Prothesenzweigen gelöst.

Zur Erfüllung der gesetzten Ziele führt die glatte oder gefaltete Gefässprothese in der Kettenbindung, welche als glatter oder verzweigter Schlauch, z.B. in zwei oder drei Zweige gestaltet ist, und welche aus synthetischen, z.B. Polyestergarnen, hergestellt ist und die an ihrer Aussen- und/oder Innenseite eine glatte oder schlingenartige Oberfläche aufweist. Die angewendeten synthetischen Fäden sind glatt oder texturiert und die Prothese ist aus gewirkten länglichen Streifen, welche miteinander entlang der Kanten verbunden sind, hergestellt. Die Verbindung ist direkt an der Fertigungsmaschine, das ist auf der zweifonturigen Kettenwirkmaschine, durchgeführt.

Das Lösungswesentliche besteht darin, dass die Gefässprothese dieses Types aus wenigstens zwei länglichen gewirkten Abschnitten rechteckiger Form besteht, die aus wenigstens einem Grundsystem glatter und/oder texturierter Fäden mit einem höheren Schrumpfeffekt und aus wenigstens einem Bauschsystem glatter und/oder texturierter Fäden mit einem niedrigeren Schrumpfeffekt gebildet sind, wobei die so erzeugten länglich gewirkten Abschnitte miteinander, was die Verbindung anbelangt, wenigstens in einem Randstäbchen des jeweiligen länglichen gewirkten Abschnittes in die Form eines übereinstimmenden Schlauchdurchmessers oder in die Form eines Schlauches mit wenigstens zwei anbindenden Zweigen, verbunden sind. Die Verbindung ist durch die gegenseitig gekreuzten Maschenfüsse, welche aus wenigstens zwei Verbindungssystemen glatter und/oder texturierter Fäden hergestellt sind, durchgeführt.

In einer Prothesenausführung ist wenigstens ein Verbindungsfadensystem entweder durch die Fäden des Grundsystems mit dem Schrumpfeffekt von 16 bis 30 % gebildet oder enthält wenigstens diese Fäden. In einer alternativen Ausführung kann wenigstens ein Verbindungsfadensystem durch die Fäden des Bauschsystems gebildet werden, mit einem Schrumpfeffekt bis zu 12 %, oder enthält wenigstens diese Fäden dieses Verbindungssystems.

In einer anderen Prothesenausführung ist wenigstens ein Verbindungsfadensystem durch Fäden des Grundsystems mit einem Schrumpfeffekt von 16 bis 30 % und den Fäden des Bauschsystems mit einem Schrumpfeffekt bis zu 12 % gebildet. Es ist ebenfalls möglich, dass wenigstens ein Verbindungsfadensystem ausser den Fäden anderer Art gerade die Fäden des Grundsystems mit dem Schrumpfeffekt von 16 bis 30 % und Fäden des Bauschsystems mit dem Schrumpfeffekt bis 12 % enthält.

Es ist vorteilhaft für die Verbindungsfadensysteme Fäden des Grundsystems und/oder Fäden des Bauschsystems , aus welchen gleichzeitig die gewirkten Maschenabschnitte hergestellt sind, welche die Prothese bilden, zu benutzen und das aus dem Grund, dass eine kompakte Durchführung der Gefässprothese mit übereinstimmenden Eigenschaften entlang des Prothesenumfanges (ganzen) erreicht wird. Selbstverständlich ist es möglich auch beliebige andere angebrachte Polyesterfäden für das Verbindungsfadensystem zu benutzen.

Die Gefässprothese kann man auch aus zusammengezwirnten oder aus gefachten Fäden des Grund- und Bauschfadensystems herstellen.

Die einzelnen gewirkten rechteckigen Abschnitte, aus welchen die Gefässprothese gebildet ist, werden in einer Doppelverbindung durch die Grundkreuzlegung oder Grundgleichlegung gewirkt.

An der Stelle, wo es zur Entzweiung der Gefässprothese kommt, das ist an der Ubergangsstelle des geraden Schlauches in die einzelnen Zweige, ist es nötig eine Prothesenaussteifung durchzuführen. Diese Ubergangsstelle wurde bisher nach Fertigwirkung der Prothese durchs Nähen ausgesteift, denn bloss durch das Wirken wurde die geforderte Prothesenschliessung nicht erreicht. An dieser Stelle war die Wanddurchlässigkeit der Gefässprothese nach Beendigung des Wirkens höher und es war nötig sie nachträglich durch einen Zusatzfaden zusammennähen, um dieses zu vermindern. Dieser Mangel wird laut der Erfindung durch Aussteifung an der gegenständlichen Prothesenübergangsstelle aus gekreuzten Maschenfüssen der Verbindungsfadensysteme behoben.

Für eine Ausscheidung der Prothesenteildrehung,eventuell der Prothesenverwindung während ihrer Anschliessung zu den anderen Gefässen bei der Operation, dient die plastische Verstärkung wenigstens eines länglichen gewirkten Abschnittes und das in den beliebigen Maschenreihen und wenigstens in einem Maschenstäbchen. Dieses längliche Reliefmuster ist aus Fäden des Grund- und/oder Bauschfadensystems, und das aus glatten und/oder texturierten Fäden, hergestellt. Das verstärkte längliche Reliefmuster ist mit Vorteil aus der gleichen Fadensorte und Fadenfarbe, wie auch die Gefässprothese gewirkt ist, hergestellt, kann jedoch auch aus Fäden unterschiedlicher Art und/oder Farbe hergestellt werden. Eine so hergestellte Leitspur dient dem Chirurgen zur Orientierung bei einer Gefässprothesenimplantation.

Die erfindungsgemässe Gefässprothese kann man für eine Spezialanwendung weiter, z.B. durch Rauhen, Schleifen, oder Stauchen ausrüsten.

Auf die Oberfläche der Gefässprothese kann man einen dünnen Polymerfilm, welcher an den einzelnen Elementarfasern haftenbleibt, auftragen, welcher jedoch die Porosität der Gefässprothese nicht vermindert. Der Zweck dieser Modifikation beruht in einer Verbesserung der Oberflächeneigenschaften der benutzten Polyesterfasern zur Prothesenherstellung, insbesonders zu ihren biokompatibilen und thromboresistenten Eigenschaften. Diese Modifikation kann man z.B. durch einen Spezialtyp von Polymerisation in einer Niederdruckhochfrequenzentladung mit Hilfe von einen dünnen Polymerfilm bilden.

Die Gefässprothese dient auch als Träger für Spezialstoffe, welche für eine Nachausrüstung der Prothese, durch welche eine Verminderung des Blutverlustes im Laufe des chirurgischen Eingriffes verfolgt wird, benutzt werden, und erfüllt die Trägerfunktion der weiteren Heilstoffe, z.B. für eine Verminderung der Blutgerinnung oder zur Gewinnung antibiotischer Wirksamkeit. Für diese Zwecke werden mit Vorteil absorbierende Stoffe auf der Eiweissbasis, z.B. Kollagen, benutzt. Vorzug dieser Stoffe beruht darin, dass sie sich nach einer bestimmten Zeit im Organismus auflösen ohne toxische Reste oder Nachwirkungen zu hinterlassen, wodurch sie ein leichtes Durchwachsen des umliegenden Gewebes in die Gefässprothesenwand unterstützen und dadurch auch ihre einwandfreie Heilung. Diese absorbierende Stoffe haften zwischen den einzelnen Elementarfasern der Fäden an und sichern eine einwandfreie Verbindung mit dem Prothesengewirke. Die Eiweissmasse hat die Fähigkeit die klinisch überprüfte Antibiotikumkombination allmählich zu deponieren, z.B. des NEOMYCINES und BACITRACINES. Gleichzeitig dient das biologisch absorbierende Material als Stoffträger mit einer antithrombogener Wirkung.

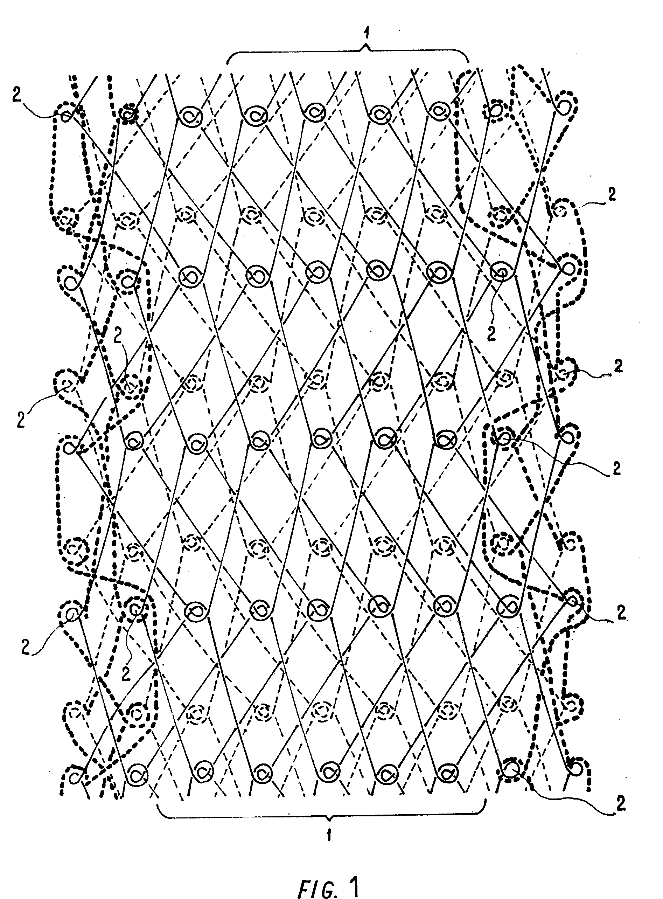

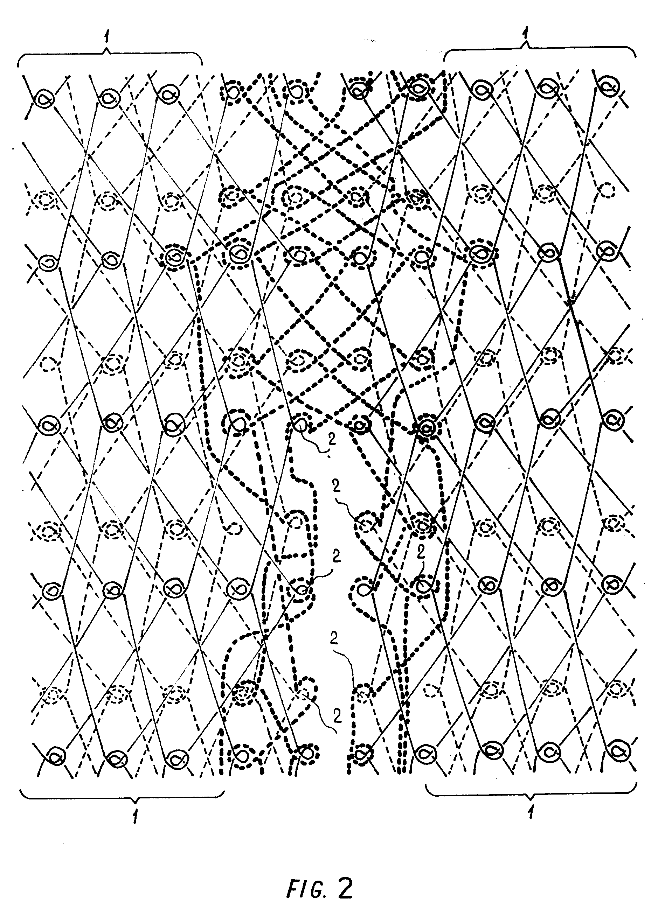

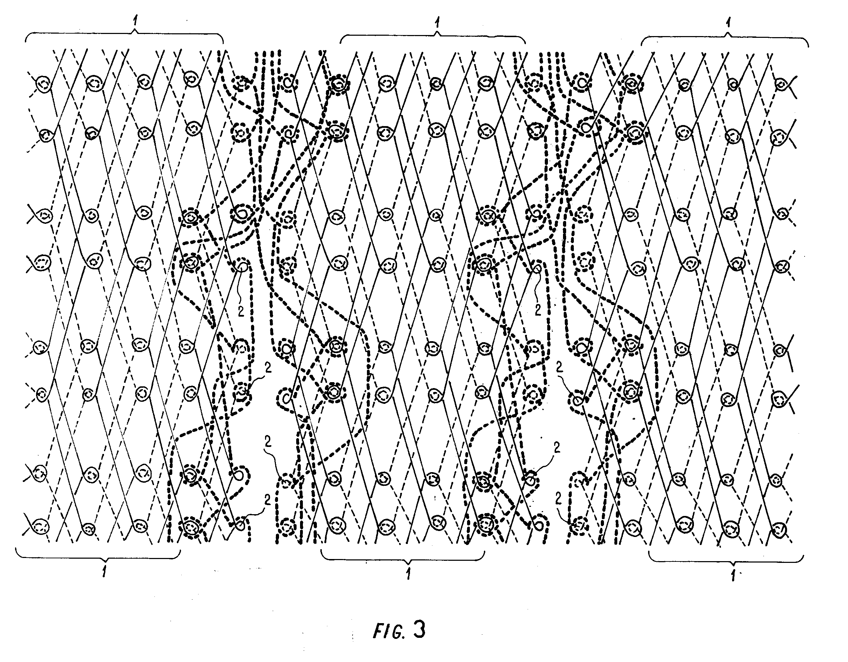

Der Fortschritt erzielt durch die Erfindung beruht darin, dass in den selbständlichen länglichen gewirkten Streifen und in den Verbindungssystemen eine Fadenkombination aus dem Grund- und Bauschsystem benutzt ist, welche Ausgangsbedingung für eine wirksame Lenkung des Blutdurchlässigkeitsgrades durch die Gefässprothesenwand sind. Die Bauschkomponente weist im Sinne der Erfindung in den gemeinsamen Maschen gegenüber der Grundkomponente einen höheren Anteil des Fadenmaterials in der Folge z.B. einer getrennten Fadenlieferung bei einer unterschiedlichen Zugkraft der Ketten auf. So ist es möglich auch eine radiale Dehnbarkeit der Gefässprothese im Einklang mit den physiologischen Bedürfnissen zu regeln. Weiter ist es nötig zu betonen, dass durch ein gegenseitiges Verbinden der länglichen gewirkten Streifen eine maximale Maschenannäherung, besonders an den Ubergangsstellen der schmalen Schläuchen in die fortschreitenden geraden Schläuche,erreicht wird. Es wurde mit Uberaschung festgestellt, dass mittels der Durchsetzung der angeführten Fadenkombinntion des Grund- und Bauschsystems gemeinsam mit der Verbindungslösung, welche hauptsächlich in einer gegenseitigen Fadenkreuzung in den beliebigen Reihen und wenigstens in einem Randmaschenstäbchen jedes länglichen gewirkten Abschnittes bei Benutzung z.B. einer umkehrbaren Legung und/oder Köperlegung beruht, eine praktische Möglichkeit der nötigen Beeinflussung der Wanddurchlässigkeit und Wandporosität der Gefässprothese gibt. Das gilt hauptsächlich für die Gefässprothesengruppe, welche mit biologisch absorbierendem Material getränkt ist. In diesen Fällen wird mit Vorteil eine höhere Wanddurchlässigkeit des Trägers benutzt, das ist des gewirkten Schlauches, was den Prozess der Prothesenheilung beschleunigt und die Qualität erhöht. Wie schon angeführt wurde, ist es vorteilhaft die Gefässprothese mit einem biologisch absorbierenden Material zu tränken, z.B. mit Kollagen, wodurch sich die Anpassungsfähigkeit und auch eine schnellere Heilung bei der Möglichkeit einer Einschränkung des Blutverlustes während der Implantation erhöht. Die biologisch absorbierende Stoffe dienen als Träger der pharmakologisch wirksamen Stoffe, welche z.B die Antibiotika oder Stoffe mit antithrombogener Eigenschaften sein können. Für die Spezialzwecke ist es angebracht die Oberfläche der Gefässprothese durch einen dünnen Polymerfilm, z.B. für die beispielhaft gewählten Prothesen mit kleinen Durchmessern zu modifizieren. Die ausführliche Erklärung des Erfindungswesens, das ist des Prinzipes der Verbindungsanordnung und Materialkombination, ist aus den beigelegten Aufzeichnungen, welche das Schema der Fadenlegung für die zweifonturige Kettenwirkmaschine darstellen, offensichtlich und das auf Fig. 1 für den Abschnitt des geraden Schlauches oder geraden Zweiges des verzweigten Schlauches einer Gefässprothese, auf Fig. 2 für die Verzweigungsstelle in zwei Zweige von einem geraden breiteren Schlauch und auf Fig. 3 für die Stelle der Verzweigung in 3 Zweige aus einem geraden breiteren Schlauch.

In Fig. 1 ist das Schema der Fadenlegung für eine zweifonturige Kettenwirkmaschine mit Benutzung von zehn Legeschienen dargestellt. Die Gefässprothese bilden zwei selbständige längliche gewirkte Maschenabschnitte 1 aus einer Kreuztrikotlegung und Kreuztuchlegung, wobei jede Masche je einen Faden des Grund- und Bauschsystems enthält. Das Grundfadensystem weist einen Schrumpfeffekt im Bereich von 16 bis 30 % auf und das Bauschfadensystem hat einen niedrigeren Schrumpfeffekt,maximal 12 %. Das Grundfadensystem bildet die Trikotbildung, das Bauschfadensystem die Tuchbildung, wobei sich die Maschenfüsse beider Bindungen gegenseitig kreuzen.

In den beiden Gewirkkanten sind die Fäden von drei Verbindungssystemen in eine Maschenform 2 eingeflechtet, welche gleichfalls Fäden des Grundsystems und/oder des Bauschsystems enthalten. Dabei kommt es zu einer länglichen Verbindung in beiden Rändern der selbständigen länglichen gewirkten Abschnitten 1 mittels Fadenkreuzung der Verbindungssysteme und das in allen Reihen und in zwei benachbarten Maschenstäbchen.

Die Prothese kann aus nur glatten Fäden erzeugt werden, das bedeutet, dass das Grund- und auch das Bauschsystem mittels dieser Fäden gebildet wird. In der alternativen Durchführung sind beide Systeme aus Texturgarn. Soweit in beide Fadensysteme, d.h. in das Grund- und auch das Bauschsystem, glatte Fäden benutzt werden, hat die Prothese den Charakter einer glatten Oberfläche. Solange Texturgarn benutzt wird, ist die Prothesenoberfläche schlingenartig.

Die Prothese kann man auch so herstellen, dass in das Grundfadensystem glatte Fäden und in das Bauschsystem Texturgarne benutzt werden. Die Anwendung von glatten und texturierten Garne kann man auch umdrehen, was bedeutet, dass die Texturgarne das Grundfadensystem bilden, während die glatten Fäden in das Bauschsystem benutzt werden.

Die Wand der Gefässprothese kann man auch mit einer Faltung versehen oder eventuell glatt, ohne einer Aussteifungswellung lassen.

Zur Erhaltung der Konstruktionskompatibilität der ganzen Gefässprothese ist es vorteilhaft in die Verbindungsfadensysteme Fäden des Grund- und/oder Bauschsystems zu benutzen. In manchen Fällen der Spezialanwendung der Gefässprothesen werden in die Verbindungsfadensysteme glatte oder Texturgarne benutzt, welche nicht schrumpfen und welche nicht zur Fadengruppe, die das Grundsystem der glatten und/oder texturierten Fäden mit einem Schrumpfeffekt von 16 bis 30 % oder in die Fadengruppe, die das Bauschsystem der glatten und/oder texturierten Fäden mit einem Schrumpfeffekt bis zu 12 % hat, gehören. In einer alternativen Ausführung der Gefässprothese ist es möglich in das Verbindungsfadensystem Fäden zu benutzen, die teilweise durch Fäden des Grundsystems mit einem Schrumpfeffekt von 16 bis 30 % und/oder durch Fäden des Bauschsystems mit dem Schrumpfeffekt bis zu 12 % gebildet sind, das bedeutet, dass die Verbindungsfadensysteme diese Fäden enthalten, und das wenigstens teilweise.

Vorteilhafte Eigenschaften der Prothese werden wie schon früher angegeben wurde, durch die Prothesenkonstruktion erreicht, in welcher die Verbindungssysteme, voll mittels Fäden des Grundsystems mit einem Schrumpfeffekt von 16 bis 30 % oder Fäden des Bauschsystems mit einem Schrumpfeffekt bis zu 12 % oder mittels einer Kombination dieser Grund- und Bauschfäden in wenigstens einem Verbindungssystem, gebildet sind.

Die Gefässprothese ist wenigstens in einem Maschenstäbchen und in den beliebigen Maschenreihen verstärkt mittels eines Reliefmusters, welches mit Vorteil aus Fäden des Grundsystems mit einem Schrumpfeffekt im Bereich von 16 bis 30 % oder aus Fäden des Bauschsystems mit dem Schrumpfeffekt bis 12 % gebildet ist. Eventuell ist das Reliefmuster aus Fäden des Grund- und Bauschsystems hergestellt. Die benutzten Faden können glatt oder texturiert sein, eventuell kann es eine Kombination glatter oder texturierter Fäden sein. Dieses Reliefmuster dient zur Orientierung des Chirurgen beim Eingriff und erleichtert das Aufkommen der Prothesenverdrehung bei der Operation zu eliminieren.

Die Prothese kann auch so hergestellt werden, dass beide längliche gewirkte Abschnitte 1 in einer Doppelverbindung mittels Kreuz- oder Gleichlegung und das aus zusammengezwirnten Fäden des Grund- und Bauschsystems erzeugt werden, wobei das Grundfadensystem einen Schrumpfeffekt von 16 bis 30 % und das Bauschsystem bis zu 12 % aufweist. Es ist auch möglich jeden länglichen gewirkten Abschnitt 1 der Prothese aus Fäden des Grund- und Bauschsystems, welche zusammen abgefacht sind, zu erzeugen. Die Fäden des Grundsystems haben einen Schrumpfeffekt von 16 bis 30 % und die Fäden des Bauschsystems bis zu 12 %.

Die Anwendung der Fäden des Grundsystems von einem Schrumpfeffekt von 16 bis 30 % und Fäden des Bauschsystems von einem Schrumpfeffekt bis zu 12 % für die Herstellung der Gefässprothese ermöglicht nach einer durchführter Prothesenfixierung, das ist bei einer geregelten Ausschrumpfung, eine geeignete eventuelle nötige Wanddurchlässigkeit der Gefässprothese zu erzielen. Das bedeutet, dass die Wanddurchlässigkeit der Gefässprothese regulierbar ist und diese Eigenschaft ist sehr vorteilhaft, denn es ist bekannt, dass die Porosität der Prothese sorgfältig kontroliert werden muss.

Auf Fig. 2 ist das Legungsschema für eine verzweigte Gefässprothese in zwei Zweige, ohne Darstellung der Prothesenränder und ihrer Verbindung dargestellt. Die Prothese bilden zwei Paare länglicher gewirkter Abschnitte 1, die nebeneinander und übereinander angebracht sind. Die einzelnen länglich gewirkten Abschnitte 1 werden wiederum in einer Doppelverbindung und das aus der Kreuztrikotlegung und einer Kreuztuchlegung hergestellt, wobei jede Gewirkmaschine je einen Faden des Grund- und Bauschsystems enthält. Zwischen beiden Zweigen der Gefässprothese ist ein freier Raum in einem Bereich von vier Reihen übereinander eingezeichnet. In diesem Reihenbereich sind beide Zweige miteinander mittels Fäden dreier Verbindungssysteme, welche gleichzeitig in einen geraden vollen Schlauchdurchmesser übergehen und welche die beiden Paare der länglichen gewirkten Abschnitte 1, die nebeneinander liegen und das im Bereich von 5 Reihen, wie in Fig. 2 angedeutet ist, verbunden. Zur Verbindung werden drei Maschenstäbchen aus jedem Paar der länglichen gewirkten Abschnitte 1 benutzt. Die Maschenfüsse der Verbindungssysteme werden gegenseitig verkreuzt. Für die Verbindungsfadensysteme werden fünf Faden des Grund- oder Bauschsystems oder eine Fadenkombination dieser Systeme benutzt.

Beide Schlauchzweige sind nach Fig. 2 im Bereich der vier Reihen verbunden, wobei es in den Verbindungen zu einer gegenseitigen Fadenkreuzung der Verbindungssysteme mittels einer umkehrbaren Legung und das im Bereich von zwei Maschenstäbchen jedes gewirkten Abschnittes 1, kommt. Die Verbindungsfadensysteme bilden die Maschen 2.

An der Ubergangsstelle des vollen Schlauches in die einzelnen Zweige wird eine Verstärkung durchgeführt und das durch die kreuzende Maschenfüsse wenigstens zweier Verbindungsfadensysteme. Diese Durchführung sichert an dieser kritischen Stelle eine übereinstimmende und dadurch auch eine nicht erhöhte Wanddurchlässigkeit der Gefässprothese, wie es der Fall der Wanddurchlässigkeit an den anderen Prothesenstellen ist.

Nach Fig. 3 ist die Gefässprothese in drei Zweige verteilt. Das Legungsschema zeigt das komplette Mittelteil der Prothese, welches aus drei selbständigen Paaren länglicher gewirkter Abschnitte 1 besteht, die an den oberen und unteren Rändern mittels des Legungsschemas bezeichnet sind. Zwischen drei Zweigen der Gefässprothese sind nicht verbundene Räume im Bereich von fünf Reihen übereinander angebracht, wobei jeder Zweig mittels Fäden der Verbindungssysteme, welche in den geraden Schlauchteil in drei Reihen übereinander mit der gegenseitigen Fadenkreuzung einer umkehrbaren Legung, begrenzt ist. In diesem Falle enthalten die Maschen 2 der Verbindungssysteme, gleich wie alle restlichen Maschen wenigstens je einen Faden des Grundsystems und wenigstens je einen Faden des Bauschsystems.

### Beispiel 1

Eine gerade und auch verzweigt gewirkte Gefässprothese wurde auf der zweifonturigen Kettenwirkmaschine erzeugt und enthält in den benutzten Fadensystemen einheitlich Polyesterseide, das bedeutet im Grund-, Bausch- und auch Verbindungssystem mit Ausnutzung der Faden die verschiedene Zugkraft der Fadenlieferung aufweisen. Es wurde die Polyesterseide Slotera 50 dtex f 24 KK NR mit einem verschiedenen Schrumpfeffekt benutzt, wobei der Fadenschrumpfeffekt des Grundsystems 22 % und des Bauschfadensystems 8 % ist. Die Zufuhrgeschwindigkeit des Bauschfadensystems ist grösser als die Zufuhrgeschwindigkeit des Grundsystems. Die benutzte Verbindung der einzelnen Paare der gewirkten länglichen Bänder ist übereinstimmend mit Fig. 2. Nach Beendigung des Wirkprozesses wurde die Prothese einer Heissfixierung ausgesetzt und das nach den Vorderungen die an sie gestellt wurden, hauptsächlich was die Durchlässigkeitsregulierung der Prothesenwand, im Bereich der Temperatur von 170 bis 190°C, anbelangt.

An der Übergangsstelle der Zweige in den vollen Schlsuchdurchmesser sind die Fäden der Verbindungssysteme auch mittels Fäden des Grund- und Bauschsystems gebildet und das wenigstens in ein Paar Reihen hintereinander zu einem Eliminierungszweck einer eventuellen Offnung, welche früher an dieser Übergangsstelle entstand, verkreuzt. Die Ausrüstung dieser Stelle ist z.B. nach Fig. 2 durchgeführt. Die Fäden des Grund- und Bauschsystems, die in den Verbindungssystemen benutzt wurden, tragen nach der Prothesenfixierung an dieser Übergangsstelle zu deren Schliessung bei.

### Beispiel 2

Eine gerade und auch verzweigte gewirkte Gefässprothese wurde auf der Maschine nach dem Beispiel 1 hergestellt, wobei eine Kombination glatter oder texturierter Polyesterseide ausgenutzt wurde und das im Grundsystem der glatten Polyesterseide 84 dtex f 48 mit einem Schrumpfeffekt von 20 % und im Bauschsystem der texturierter Polyesterseide Slotera 84 dtex f 48 KK NR mit einem Schrumpfeffekt von 7 %. In die Verbindungssysteme wurde Polyesterseide beider Typen benutzt. Nach der Heissfixierung bei 180°C war die Wanddurchlässigkeit der Prothese auf der Fläche eines Quadratzentimeters bei einem Überdruck von 16 kPa 6 Liter pro Minute. Diese Prothese wurde mit Kollagen, welcher aus Rindsspaltleder gewonnen wird, getränkt, wodurch die Wanddurchlässigkeit des Implantats bei dem höher angegebenen Überdruck auf den Nullwert herabgesetzt wurde.

### Beispiel 3

Die gewirkte gerade und auch verzweigte Gefässprothese wurde auf der Maschine nach Beispiel 1 erzeugt, wo zur Erhöhung der Bauschigkeit und Verminderung der Gefässprothesendurchlässigkeit die Materialkombination glatter Polyesterseide 84 dtex f 48 mit einem Schrumpfeffekt von 21 % in dem Grundsystem der Kettfäden und der texturierten Polyesterseide Slotera 84 dtex f 48 KK NR mit dem Schrumpfeffekt von 8 % im Bauschfadensystem ausgenutzt wurde. In die Systeme der Verbindungskettfäden wurde Polyesterseide 50 dtex f 24 benutzt.

### Beispiel 4

Die gerade und auch versweigt gewirkte Gefässprothese wurde auf der Maschine nach dem Beispiel 1 hergestellt, wo das Material glatte Polyesterseide Tetoron 75 dtex f 36 mit einem Schrumpfeffekt von 22 % im Grundsystem der Kettfäden und texturierte Polyesterseide 110 dtex f 48 KK NR mit einem Schrumpfeffekt von 8 % in das Bauschsystem der Kettfäden benutzt wurde. In die Verbindungssysteme wurde die glatte Polyesterseide Slotera f 38 mit dem Schrumpfeffekt 8 % benutzt.

### Beispiel 5

Die gerade und auch verzweigt gewirkte Gefässprothese wurde auf der zweifonturigen Kettenwirkmaschine hergestellt. Das Grundsystem der Kettfäden bildete die Polyesterseide 84 dtex f 48 Slotera-glatte mit dem Schrumpfeffekt von 21 % und das Bauschfadensystem bildete die Polyesterseide 84 dtex Slotera-glatte mit dem Schrumpfeffekt von 8 %,welche das Fadensystem Slotera 50 dtex f 24 KK NR verbinden.

Eine weitere Appretur in allen angegebenen Fällen der Gefässprothesenherstellung beruht in der eingeführten Technologie und umfasst das Waschen, Spülen, Extraktion in chlorierten Derivaten, Fixierung der Masse und formen nach den vorbereiteten Formen und Sterilisation.Es wurde auch eine Prothesenfaltung nach irdendeiner bekannten Technologie durchgeführt.

Die Prothesenoberfläche kann man auch durch Aufrauhen, Schleifen, Stauchen appretieren, je nach den Anforderungen die an das Erzeugnis gestellt sind. Mit Vorteil werden in das biologisch absorbierende Material der Prothese pharmakologisch wirksame Stoffe, z.B. Antibiotika, Stoffe mit antithrombogener Wirkung, zugegeben.

## Patentansprüche

1. Gewirkte glatte oder gefaltete Gefässprothese aus glatten oder texturierten synthetischen Fäden, z.B. Polyesterfasern, in der Form eines glatten oder verzweigten Schlauches von geforderten Durchmessern, mit einer glatten oder schlingenartigen Oberfläche an der Aussen- und/oder Innenseite der Prothese, die aus länglichen gewirkten Striefen, welche miteinander längs der Ränder verbunden sind in der Kettenbindung gebildet ist, dadurch gekennzeichnet, dass die Gefässprothese aus wenigstens zwei länglichen gewirkten Abschnitten (1) rechteckiger Form besteht, die aus wenigstens einem Grundsystem glatter und/oder texturierter Fäden mit einem höheren Schrumpfeffekt und aus wenigstens einem Bauschsystem glatter und/oder texturierter Fäden mit einem niedrigeren Schrumpfeffekt gebildet sind, wobei die so erzeugten länglich gewirkten Abschnitte (1) miteinander, was die Verbindung anbelangt, wenigstens in einem Randstäbchen jedes länglichen gewirkten Abschnittes (1) in die Form eines übereinstimmenden Schlauchdurchmessers oder in die Form eines Schlauches mit wenigstens zwei anbindenden Zweigen mittels gegenseitig gekreuzter Maschenfüsse (2), die aus wenigstens zwei Verbindungssystemen glatter und/oder texturierter Fäden gebildet sind, verbunden sind.

2. Gewirkte glatte oder gefaltete Gefässprothese, nach Anspruch 1, dadurch gekennzeichnet, dass das Grundsystem der glatten und/oder texturierten Fäden einen Schrumpfeffekt im Bereich von 16 bis 30 % aufweist, während das Bauschsystem der glatten und/oder texturierten Fäden einen Schrumpfeffekt bis 12 % aufweist.

3. Gewirkte glatte oder gefaltete Gefässprothese, nach Anspruch 1, dadurch gekennzeichnet, dass wenigstens ein Verbindungsfadensystem durch Fäden des Grundsystems mit einem Schrumpfeffekt von 16 bis 30 % gebildet ist.

4. Gewirkte glatte oder gefaltete Gefässprothese, nach Anspruch 1, dadurch gekennzeichnet, dass wenigstens ein Verbindungsfadensystem durch Fäden des Bauschsystems mit dem Schrumpfeffekt bis 12 % gebildet ist.

5. Gewirkte glatte oder gefaltete Gefässprothese, nach Anspruch 1, dadurch gekennzeichnet, dass wenigstens ein Verbindungsfadensystem teilweise Fäden des Grundsystems mit dem Schrumpfeffekt 16 bis 30 % enthält.

6. Gewirkte glatte oder gefaltete Gefässprothese, nach Anspruch 1, dadurch gekennzeichnet, dass wenigstens ein Verbindungsfadensystem teilweise Fäden des Bauschsystems mit dem Schrumpfeffekt bis 12 % enthält.

7. Gewirkte glatte oder gefaltete Gefässprothese, nach Anspruch 1,dadurch gekennzeichnet, dass wenigstens ein Verbindungsfadensystem teilweise Fäden des Grundsystems mit dem Schrumpfeffekt 16 bis 30 % und Fäden des Bauschsystems mit dem Schrumpfeffekt bis 12 % enthält.

8. Gewirkte glatte oder gefaltete Gefässprothese, nach Anspruch 1, dadurch gekennzeichnet, dass wenigstens ein Verbindungsfadensystem durch Fäden des Grundsystems mit einem Schrumpfeffekt von 16 bis 30 % und Fäden des Bauschsystems mit dem Schrumpfeffekt bis 12 % gebildet ist.

9. Gewirkte glatte oder gefaltete Gefässprothese, nach Anspruch 1, dadurch gekennzeichnet, dass die Fäden des Grundaystems mit den Fäden des Bauschsystems zusammengezwirnt sind.

10. Gewirkte glatte oder gefaltete Gefässprothese, nach Anspruch 1, dadurch gekennzeichnet, dass die Fäden des Grundsystems mit den Fäden des Bauschsystems gefachtet sind.

11. Gewirkte glatte oder gefaltete Gefässprothese, nach Anspruch 1, dadurch gekennzeichnet, dass die länglichen gewirkten Abschnitte (1) in einer Doppelbindung durch die Grundkreuzlegung gebildet sind.

12. Gewirkte glatte oder gefaltete Gefässprothese, nach Anspruch 1, dadurch gekennzeichnet, dass die länglichen gewirkten Abschnitte (1) in einer Doppelbindung durch die Grundgleichlegung gebildet sind.

13. Gewirkte glatte oder gefaltete Gefässprothese, nach Anspruch 1, dadurch gekennzeichnet, dass an der Ubergangsstelle des geraden Schlauches in die einzelnen Zweige, eine Prothesenaussteifung aus gekreuzten Maschenfüssen (2) der Verbindungsfadensysteme durchgeführt wurde.

14. Gewirkte glatte oder gefaltete Gefässprothese, nach Anspruch 1, dadurch gekennzeichnet, dass wenigstens ein länglicher gewirkter Abschnitt (1) in den beliebigen Maschenreihen und in wenigstens einem Maschenstäbchen mittels eines Reliefmusters verstärkt ist.

15. Gewirkte glatte oder gefaltete Gefässprothese, nach Anspruch 14, dadurch gekennzeichnet, dass das längliche Reliefmuster aus Fäden des Grund- und/oder Bauschsystems und das aus glatten und/oder texturierten Fäden gebildet ist.

16. Gewirkte glatte oder gefaltete Gefässprothese, nach Anspruch 1, dadurch gekennzeichnet, dass die Gefässprothese durch Aufrauhen, Schleifen, Stauchen oberflächlich appretiert wird.

17. Gewirkte glatte oder gefaltete Gefässprothese, nach Anspruch 1, dadurch gekennzeichnet, dass die Gefässprothese durch einen dünnen Polymerfilm oberflächlich modifiziert wird.

18. Gewirkte glatte oder gefaltete Gefässprothese, nach Anspruch 1, dadurch gekennzeichnet, dass die Gefässprothese durch einen biologisch absorbierenden Stoff z.B. Kollagen, getränkt ist.

19. Gewirkte glatte oder gefaltete Gefässprothese, nach Anspruch 18, dadurch gekennzeichnet, dass der biologisch absorbierende Stoff pharmakologisch wirksame Stoffe enthält, z.B. Antibiotika und Stoffe mit einer antithrombogenen Wirkung.

## Claims

1. A non-crimped or crimped knitted vascular prosthesis made of flat or texturized synthetic threads, for example polyester filaments, in the form of a straight or bifurcated hose of desired diameters with flat or looped inner and/or outer surfaces, the prosthesis consisting of longitudinal warp-knitted sections joined together along the edges, wherein said prosthesis consists of at least two longitudinal warp-knitted sections (1) of rectangular form comprising at least one basic system of flat and/or texturized threads with a higher shrinkage and at least one bulky system of flat and/or texturized threads with a lower shrinkage, said longitudinal warp-knitted sections (1) manufactured in this manner being joined in a warp-knitted structure along at least one selvedge wale of each longitudinal warp-knitted section (1) into a shape of a hose having a uniform diameter, or into a shape of a hose branching into at least two branches, by means of crossed connecting stitch loops (2) formed of at least two connecting systems of flat and/or texturized threads.

2. A non-crimped or crimped knitted vascular prosthesis as claimed in claim 1., wherein the basic system of flat and/or texturized threads has a shrinkage in the range from 16 to 30 %, while the bulky system of the flat and/or texturized threads has a shrinkage of up to 12 %.

3. A non-crimped or crimped knitted vascular prosthesis as claimed in claim 1., wherein at least one connecting system of threads consists of threads of the basic system with a shrinkage of 16 to 30 %.

4. A non-crimped or crimped knitted vascular prosthesis as claimed in claim 1., wherein at least one connecting system of threads consists of threads of the bulky system with a shrinkage of up to 12 %.

5. A non-crimped or crimped knitted vascular prosthesis as claimed in claim 1., wherein at least one connecting system of threads partly contains threads of the basic system with a shrinkage of 16 to 30 %.

6. A non-crimped or crimped knitted vascular prosthesis as claimed in claim 1., wherein at least one connecting system of threads partly contains threads of the bulky system with a shrinkage of up to 12 %.

7. A non-crimped or crimped knitted vascular prosthesis as claimed in claim 1., wherein at least one connecting system of threads partly contains threads of the basic system with a shrinkage of 16 to 30 %, and threads of the bulky system with a shrinkage of up to 12 %.

8. A non-crimped or crimped knitted vascular prosthesis as claimed in claim 1., wherein at least one connecting system of threads consists of threads of the basic system with a shrinkage of 16 to 30 % and threads of the bulky system with a shrinkage of up to 12 %.

9. A non-crimped or crimped knitted vascular prosthesis as claimed in claim 1., wherein threads of the basic system are plied together with threads of the bulky system.

10. A non-crimped or crimped knitted vascular prosthesis as claimed in claim 1., wherein threads of the basic system are doubled together with threads of the bulky system.

11. A non-crimped or crimped knitted vascular prosthesis as claimed in claim 1., wherein longitudinal knitted sections (1) are made in a double structure with a basic lapping movement in opposite directions.

12. A non-crimped Or crimped knitted vascular prosthesis as claimed in claim 1., wherein longitudinal knitted sections (1) are made in a double structure with a unidirectional basic lapping movement.

13. A non-crimped or crimped knitted vascular prosthesis as claimed in claim 1., wherein a reinforcement of the prosthesis with crossed connecting stitch loops (2) of the connecting systems of threads is effected at the transition of straight hose into branches.

14. A non-crimped or crimped knitted vascular prosthesis as claimed in claim 1., wherein at least one longitudinal knitted section (1) is reinforced with a longitudinal relief pattern in optional number of stitch courses and at least in one wale.

15. A non-crimped or crimped knitted vascular prosthesis as claimed in claim 14., wherein the longitudinal relief pattern is formed of threads of the basic and/or bulky system, namely of flat and/or texturized threads.

16. A non-crimped or crimped knitted vascular prosthesis as claimed in claim 1., wherein its surface is finished by raising, grinding, stuffing.

17. A non-crimped or crimped knitted vascular prosthesis as claimed in claim 1., wherein a thin polymeric film is applied to its surface.

18. A non-crimped or crimped knitted vascular prosthesis as claimed in claim 1., wherein it is impregnated with a biologically absorbable material, e.g. collagen.

19. A non-crimped or crimped knitted vascular prosthesis as claimed in claim 18., wherein the biologically absorbable material contains pharmacologically effective substances, e.g. antibiotics and substances with antithrombogenic effect.

## Revendications

1. Prothèse vasculaire tricotée, lisse ou plissé, fabriquée des fils synthétiques plats ou texturisés, par exemple de polyester, en forme d'une tube droite ou branchée d'un diamètre voulu, avec une surface lisse ou à boucles sur la face ou sur l'envers de la prothèse constituée par des parties longitudinales tricotées liées le long des lisières en armure par chaîne, caractérisée en ce qu'elle consiste le moins de deux parties longitudinales tricotées (1) d'une forme rectangulaire qui sont constituées le moins d'un système principal des fils plats et/ou texturisés d'une rétractibilité plus haute et le moins d'un système volumineux des fils plats et/ou texturisés d'une rétractabilité plus basse étant donnée que les parties longitudinales tricotées (1), quant à leur liaison, sont assemblés le moins dans une colonne de mailles de bord de chaque partie longitudinale tricotée (1) en forme d'une tube d'un diamètre identique ou en forme d une tube le moins avec deux branches attachées à l'aide des mailles croisées (2), formées le moins de deux systèmes de liaison des fils plats et/ou fils texturisés.

2. Prothèse vasculaire tricotée, lisse ou plissée, selon la revendication 1., caractérisée en ce que le système principal des fils plats et/ou texturisés présente une rétractabilité dans l'étendue de 16 jusqu' à 30 pour cent tandis que le système volumineux des fils plats et/ou texturisés présente une rétractabilité au maximum de 12 pour cent.

3. Prothèse vasculaire tricotée, lisse ou plissée, selon la revendication 1., caractérisée en ce que le moins un système est constitué par les fils de système principal d'une rétractabilité de 16 jusqu' à 30 pour cent.

4. Prothèse vasculaire tricotée, lisse ou plissée, selon la revendication 1., caractérisée en ce que le moins un système de liaison est constitué par les fils de système volumineux avec une rétractabilité au maximum de 12 pour cent.

5. Prothèse vasculaire tricotée, lisse ou plissée, selon la revendication 1., caractérisée en ce que le moins un système de liaison contient partiellement des fils du système principal d'une rétractabilité de 16 jusqu' à 30 pour cent.

6. Prothèse vasculaire tricotée, lisse ou plissée, selon la revendication 1., caractérisée en ce que le moins un système de liaison contient partiellement des fils du système volumineux d'une rétractabilité au maximum de 12 pour cent.

7. Prothèse vasculaire tricotée, lisse ou plissée, selon la revendication 1., caractérisée en ce que le moins un système de liaison contient partiellement des fils du système principal d'une rétractabilité de 16 jusqu' à 30 pour cent et des fils du système volumineux d'une rétractabilité au maximum de 12 pour cent.

8. Prothèse vasculaire tricotée, lisse ou plissée, selon la revendication 1., caractérisée en ce que le moins un système de liaison est constitué par les fils du système principal d'une rétractabilité de 16 jusqu' à 30 pour cent et par les fils du système volumineux d'une rétractabilité au maximum de 12 pour cent.

9. Prothèse vasculaire tricotée, lisse ou plissée, selon la revendication 1., caractérisée en ce que les fils du système principal sont assemblés par retordage avec les fils du système volumineux.

10. Prothèse vasculaire tricotée, lisse ou plissée, selon la revendication 1., caractérisée en ce que les fils du système principal sont assemblés avec les fils du système volumineux par doublage.

11. Prothèse vasculaire tricotée, lisse ou plissée, selon la revendication 1., caractérisée en ce que les parties longitudinales tricotées (1) sont constituées d une armure double en résultat d'une jetée de base en sens contraire.

12. Prothèse vasculaire tricotée, lisse ou plissée, selon la revendication 1., caractérisée en ce que les parties tricotées longitudinales (1) sont constituées d une jetée de base unidirectionnelle.

13. Prothèse vasculaire tricotée, lisse ou plissée, selon la revendication 1., caractérisée en ce qu'elle est renforcée dans le point de branchement de la tube par les boucles d'aiguille croiésées (2) en fils des systèmes de liaison.

14. Prothèse vasculaire tricotée, lisse ou plissée, selon la revendication 1., caractérisée en ce que le moins une des parties tricotées longitudinales (1) est renforcée en rangées des mailles quelconques et le moins en une colonne des mailles renforcée d'un dessin longitudinal en relief.

15. Prothèse vasculaire tricotée, lisse ou plissée, selon la revendication 14., caractérisée en ce que le dessin longitudinal en relief est constitué des fils du système de base et/ou du système volumineux, à savoir des fils plats et/ou texturisés.

16. Prothèse vasculaire tricotée, lisse ou plissée, selon la revendication 1., caractérisée en ce que la prothèse est traités sur sa surface par grattage, émerisage et écrasement.

17. Prothèse vasculaire tricotée, lisse ou plissée, selon la revendication 1., caractérisée en ce que la surface de la prothèse est modifiée par l'enduction d'un film de polymère d'une faible épaisseur.

18. Prothèse vasculaire tricotée, lisse ou plissée, selon la revendication 1., caractérisée en ce que la prothèse est imprégnée d'une substance d'une degradabilité biologique, par exemple de collagène.

19. Prothèse vasculaire tricotée, lisse ou plissée, selon la revendication 18., caractérisée en ce que la substance d'une degradabilité biologique contient des substances d'une éfficacité pharmacologique, par exemple des substances antibiotiques ou des substances d'une éfficacité antithrombogène.
